# EUROPÄISCHE PATENTSCHRIFT

(11) **EP 2 986 345 B1**
(45) Veröffentlichungstag und Bekanntmachung des Hinweises auf die Patenterteilung: **14.06.2017**
(21) Anmeldenummer: 14718513.6
(22) Anmeldetag: 16.04.2014
(51) Int. Cl.: A61K 8/41, A61K 8/44, A61K 8/06, A61K 8/31, A61K 8/36, A61Q 19/10, A61Q 5/02, A61K 8/37, A61Q 1/02, A61K 8/60, A61K 8/89, A61K 8/92, A61Q 19/00

(54) **ZUSAMMENSETZUNG ENTHALTEND ÖLKÖRPER, FETTSÄUREN, AMINOSÄURETENSIDE UND N-METHYL-N-ACYLGLUCAMINE**
COMPOSITION COMPRISING OIL, FATTY ACIDS, SUPERFICIALLY ACTIVE AGENTS ON THE BASIS OF AMINO ACIDS, AND N-METHYL-N-ACYLGLUCAMINES
COMPOSITION CONTENANT HUILE, ACIDES GRAS, LES TENSIOACTIFS SUR LA BASE D'AMINOACIDES, ET N-MÉTHYL-N-ACYLGLUCAMINES.

(30) Priorität: 20.04.2013 DE 102013006879
(43) Veröffentlichungstag der Anmeldung: 24.02.2016
(73) Patentinhaber: Clariant International Ltd, 4132 Muttenz (CH)
(72) Erfinder: KLUG, Peter, 63762 Grossostheim (DE); MILDNER, Carina, 65366 Geisenheim (DE); BAUER, Martin, 60329 Frankfurt am Main (DE)
(74) Vertreter: Holmes, Rosalind
(86) Internationale Anmeldenummer: PCT/EP2014/001022
(87) Internationale Veröffentlichungsnummer: WO 2014/170025

(56) Entgegenhaltungen:
- EP-A1- 1 716 842
- WO-A1-99/51716
- US-B1- 6 903 057

## Beschreibung

Die Erfindung betrifft eine Zusammensetzung, enthaltend Ölkörper, Fettsäuren, Aminosäuretenside und N-Methyl-N-acylglucamine. Ferner betrifft die Erfindung die Verwendung der Zusammensetzung zur Behandlung oder Pflege der Haut oder Haare, beispielsweise als Shampoo, Gesichtsreiniger, Flüssigreiniger oder Duschbad.

Die Produktion flüssiger Produkte auf dem Kosmetik und Waschmittelsektor nimmt ständig zu. Speziell im Bereich der Körperreinigungsmittel sind es flüssige Zusammensetzungen wie Haarshampoos, Schaumbäder und Duschbäder, die in den letzten Jahren zunehmend an Bedeutung gewonnen haben.

Solche Zusammensetzungen enthalten neben Wasser und Tensiden häufig einen Ölkörper, beispielsweise natürliche Öle oder Paraffine. In Kombination mit Fettsäuren wird damit ein angenehmes Hautgefühl erzeugt. Ölkörper, insbesondere in Kombination mit Fettsäuren stellen jedoch eine Herausforderung für die Entwicklung eines geeigneten Tensidsystems dar.

Voraussetzung für eine gute Tensidrezeptur ist eine gute Lagerstabilität. Die Zusammen-setzung darf bei Temperaturschwankungen nicht eintrüben oder Absetzungen bilden und sollte eine Viskosität aufweisen, die dem jeweiligen Anwendungszweck angepasst werden kann. Das Viskositätsprofil der Rezeptur soll weiterhin über einen weiten Temperaturbereich konstant sein, damit die Formulierung unabhängig von der Umgebungstemperatur handhabbar ist. Somit ist die Viskosität ein Qualitätskriterium. Der Grad der Viskosität hängt von dem Tensidsystem, dem Elektrolytzusatz und auch vom Gehalt der Tensidformulierung an Ölkörpern ab. Gibt man Ölkörper in tensidhaltige Formulierungen, so sieht man im Allgemeinen einen drastischen Viskositätsabfall. Es bilden sich Emulsionen, die in der Praxis mit verdickenden Polymeren, wie z. B. Polyacrylaten, Xanthan Gum oder Stärkederivaten wie Natrium Hydroxypropyl-Stärkephosphate verdickt werden müssen, um eine ausreichende Stabilität zu erzielen.

Die Aufgabe der Erfindung liegt somit darin, verbesserte ölhaltige Zusammensetzungen bereitzustellen, die stabilere Emulsionen erzeugen, den Bedarf an Verdickerpolymeren reduzieren und temperaturstabilere Rezepturen US6903057B1 offenbart flüssige Reinigungszusammensetzungen für die Körperwäsche umfassend (1) 2 bis 30 Gew. % eines Tensids, das aus der Gruppe ausgewählt ist, die aus anionischen Tensiden, nichtionischen Tensiden, amphoteren Tensiden, kationischen Tensiden oder Gemischen davon besteht; (2) ein Strukturierungssystem umfassend etwa 6 bis 30 % modifizierte oder nicht-modifizierte Stärkekörnchen; und (3) 0 bis 30 % Vorteilsmittel. US6903057B1 offenbart Beispiele umfassend Natriumcocoylmethylltaurat und Kaliumcocoylglycinat. Die Verwendung von N-Polyhydroxyalkylfettsäureamiden in kosmetischen Zusammensetzungen und Waschmitteln ist bekannt.

N-Methyl-N-acylglucamine mit C₁₂- und C₁₄-Acylgruppen und Fettsäureethersulfaten oder Fettsäuresulfaten sind in der WO 92/06158 und WO 92/06162 zur Verwendung in Spül- und Reinigungsmitteln beschrieben.

Die WO 98/56496 betrifft eine Tensidzusammensetzung mit verbesserter Schaumstabilität. Die Tensidzusammensetzung enthält: (a) von ungefähr 1 bis ungefähr 40 Gew.-% eines Zuckertensids; (b) von ungefähr 1 bis ungefähr 40 Gew.-% eines anionischen Tensids; (c) von ungefähr 0,11 bis ungefähr 10 Gew.-% eines Amphoacetats; und (d) Wasser, wobei sich die Gewichtsangaben auf das Gewicht der Zusammensetzung beziehen.

In der EP-A 0 285 768 ist die Verwendung von N-Polyhydroxyalkylfettsäureamiden als Verdickungsmittel für flüssige wässrige Tensidsysteme beschrieben.

Aus keinem der genannten Dokumente lässt sich ableiten, dass N-Methyl-N-acylglucamine in Kombination mit bestimmten weiteren Tensiden sich insbesondere zum Einsatz in Ölkörper und Fettsäuren enthaltenden Zusammensetzungen eignen.

Es wurde nun gefunden, dass Zusammensetzungen, die neben einem Ölkörper und Fettsäure(n) eine Kombination aus einem Aminosäuretensid und einem Glucamid enthalten, die genannte Aufgabe lösen.

Demgemäß wird eine Zusammensetzung bereitgestellt, enthaltend:
(A) mindestens ein N-Acyl-aminosäuretensid als Komponente A, wobei Komponente A aus mindestens einer C₈-C₂₂-acylierten Aminosäure besteht, und ausgewählt ist aus der Gruppe bestehend aus Acylglycinat, Acylaspartat, Acylglutamat, Acylsarkosinat, deren Salze und Mischungen davon,
(B) mindestens ein N-Methyl-N-acylglucamin als Komponente B,
(C) mindestens eine C₈-C₂₂ Fettsäure oder einem Fettsäuresalz als Komponente C,
(D) mindestens einen Ölkorper als Komponente D,
(E) optional mindestens einen Acylisethionat als Komponente E,
(F) optional mindestens ein anionisches, sulfoniertes Tensid als Komponente F,
(G) optional mindestens ein Betaintensid als Komponente G,
(H) gegebenenfalls ein oder mehrere Additiven als Komponente H und
(I) Wasser.

Die erfindungsgemäße Zusammensetzung weist vorteilhafterweise eine verbesserte Emulsionsstabilität und ein verbessertes Viskositätsverhalten bei hoher Temperatur und damit eine verbesserte Lagerstabilität auf.

Erfindungsgemäß bevorzugt ist eine Zusammensetzung enthaltend:
(A) mindestens eine N-Acyl-aminosäuretensid als Komponente A, wobei Komponente A aus mindestens einer C₈-C₂₂-acylierten Aminosäure besteht, und ausgewählt ist aus der Gruppe bestehend aus Acylglycinat, Acylaspartat, Acylglutamat, Acylsarkosinat, deren Salze und Mischungen davon,
(B) mindestens ein N-Methyl-N-acylglucamin als Komponente B,
(C) mindestens eine C₁₂-C₂₂ Fettsäure oder ein Fettsäuresalz als Komponente C,
(D) mindestens ein Ölkorper als Komponente D,
(E) mindestens ein Acylisethionat als Komponente E,
(F) optional ein oder mehrere anionische, sulfonierte Tensid als Komponente F,
(G) optional ein oder mehrere Betaintenside als Komponente G,
(H) optional ein oder mehrere Additive als Komponente H und
(I) Wasser.

Bevorzugt ist auch eine Zusammensetzung, welche neben den Komponenten A, B, C und D die Komponente F enthält.

Bevorzugt ist auch eine Zusammensetzung, welche neben den Komponenten A, B, C und D die Komponente G enthält.

Bevorzugt ist auch eine Zusammensetzung, welche neben den Komponenten A, B, C und D die Komponente H enthält.

Bevorzugt ist auch eine Zusammensetzung, welche neben den Komponenten A, B, C und D die Komponenten E und F enthält.

Bevorzugt ist auch eine Zusammensetzung, welche neben den Komponenten A, B, C und D die Komponenten E und G enthält.

Bevorzugt ist auch eine Zusammensetzung, welche neben den Komponenten A, B, C und D die Komponenten E und H enthält.

Bevorzugt ist auch eine Zusammensetzung, welche neben den Komponenten A, B, C und D die Komponenten F und G enthält.

Bevorzugt ist auch eine Zusammensetzung, welche neben den Komponenten A, B, C und D die Komponenten F und H enthält.

Bevorzugt ist auch eine Zusammensetzung, welche neben den Komponenten A, B, C und D die Komponenten G und H enthält.

Bevorzugt ist auch eine Zusammensetzung, welche neben den Komponenten A, B, C und D die Komponenten E, F und G enthält.

Bevorzugt ist auch eine Zusammensetzung, welche neben den Komponenten A, B, C und D die Komponenten E; F und H enthält.

Bevorzugt ist auch eine Zusammensetzung, welche neben den Komponenten A, B, C und D die Komponenten E; G und H enthält.

Bevorzugt ist auch eine Zusammensetzung, welche neben den Komponenten A, B, C und D die Komponenten F, G und H enthält.

Bevorzugt ist auch eine Zusammensetzung, welche neben den Komponenten A, B, C und D die Komponenten E, F, G und H enthält.

Weiterhin bevorzugt ist eine Zusammensetzung enthaltend:

| | | |
|---|---|---|
| (A) | 0,5 - 10 | Gew.-% der Komponente A, |
| (B) | 0,5 - 10 | Gew.-% der Komponente B, |
| (C) | 0,5 - 5,0 | Gew.-% der Komponente C |
| (D) | 0,5 - 8,0 | Gew.-% der Komponente D, |
| (E) | 0 bis 8,0 | Gew-% der Komponente E, |
| (F) | 0 - 10 | Gew.-% der Komponente F, |
| (G) | 0 - 8 | Gew.-% der Komponente G, |
| (H) | 0 - 15 | Gew.-% eines oder mehrerer Additive H |
| (I) | 74 - 98 | Gew-% Wasser |

wobei die Summe der Komponenten A bis I 100 Gew.-% ergibt.

Weiterhin bevorzugt sind Zusammensetzungen, enthaltend:

| | |
|---|---|
| (A) | 1 - 6,0 Gew.-% der Komponente A, bevorzugt 2,0 bis 4,0 Gew.-% |
| (B) | 1 - 5,0 Gew.-% der Komponente B, bevorzugt 2,0 bis 4,0 Gew.-% |
| (C) | 1 - 4,0 Gew.-% der Komponente C, bevorzug 1,5 bis 3,0 Gew.-% |
| (D) | 1 - 5,0 Gew.-% der Komponente D, bevorzugt 1,5 bis 3,0 Gew.-% |
| (E) | 0 bzw. 0,5 bis 2,0 Gew-% der Komponente E, |
| (F) | 0 bzw. 1 - 5 Gew.-% der Komponente F, bevorzugt 0,5 bis 3,0 Gew.-% |
| (G) | 0 bzw. 0,5 - 3 Gew.-% der Komponente G, |
| (H) | 0 bzw. 5 - 10 Gew.-% eines oder mehrerer Additive H und |
| (I) | 40 - 98 bzw. 40 - 89 Gew-% Wasser |

wobei die Summe der Komponenten A bis I 100 Gew.-% ergibt.

Der als bzw. gekennzeichnete Wert bezeichnet die bevorzugte Untergrenze falls die Komponente enthalten ist. Im Falle von Wasser (I) bezeichnet der Wert "bzw." die Grenzen, falls alle Komponenten vorhanden sind.

Für alle genannten Zusammensetzungen gilt, dass sie vorzugsweise aus den Komponenten (A) bis (I) bestehen.

### (A)

Die Zusammensetzung enthält:
(A) mindestens eine N-Acyl-aminosäuretensid als Komponente A, wobei Komponente A aus mindestens einer C₈-C₂₂-acylierten Aminosäure besteht, und ausgewählt ist aus der Gruppe bestehend aus Acylglycinat, Acylaspartat, Acylglutamat, Acylsarkosinat, deren Salze und Mischungen davon.

Im Rahmen einer bevorzugten Ausführungsform ist die Komponente A ausgewählt ist aus der Gruppe bestehend aus Acylglycinat, Acylaspartat, Acylglutamat, Acylsarkosinat oder Mischungen daraus.

Bevorzugt sind Acylglycinate, Acylaspartate, Acylglutamate und Acylsarkosinate, besonders Natriumcocoylglycinat, Kaliumcocoylglycinat, Natriumlauroylglycinat, Kaliumlauroylglycinat, Natriumcocoylglutamat, Natriumlauroylglutamat, Natriumcocoylaspartat, Natriumlauroylaspartat und Natriumlauroylsarkosinat.

Im Rahmen einer bevorzugten Ausführungsform besteht die Komponente A aus mindestens eines N-alkylierten Derivates der C₈-C₂₂-acylierten Aminosäure. Bevorzugt sind die entsprechenden Lauroyl- oder Cocoylderivate der Aminosäuren.

Besonders bevorzugt wird Na-Acylglycinat und K-Acylglycinat. Besonders bevorzugt sind auch Na-Cocoylglycinat und Na-Lauroylglycinat.

### (B)

Weitere Begriffe für N-Methyl-N-acylglucamin sind N-Methyl-N-1-Desoxysorbitol-Fettsäureamid, N-Acyl-N-methyl-glucamin, Glucamid oder N-Methyl-N-alkylglucamid.

Als bevorzugte Ausführungsform werden als Komponente (B) N-Methyl-N-acylglucamine und deren Mischungen (auch als N-Methyl-N-1-Desoxisorbityl-Fettsäureamide bekannt) verwendet, die einen gesättigten oder ungesättigten, geradkettigen oder verzweigten C₈-C₂₂, bevorzugt einen geradkettigen, gesättigten oder ungesättigten C₁₂-C₁₈ Acylrest tragen. Dabei entspricht N-Methyl-N-acylglucamin der Formel (I), wobei R^{a} ein C₇-C₂₁-Kohlenwasserstoffrest ist.

Insbesondere bevorzugt als Komponente (B) sind N-Methyl-N-acylglucamine der Formel (I), wobei der Acylrest R^{a}CO abgeleitet ist von der Octansäure, Decansäure, Laurinsäure, der Myristinsäure, der Palmitinsäure, der Stearinsäure, der Ölsäure, der Linolsäure oder der Linolensäure:

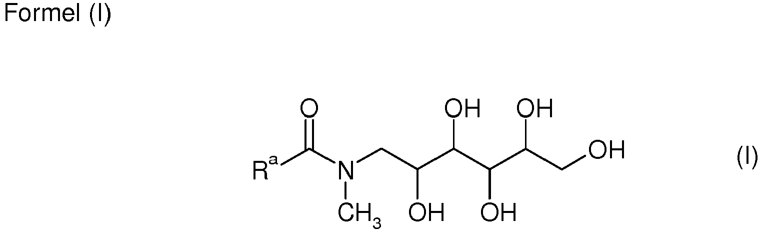

Besonders bevorzugt liegt der Anteil an N-Methyl-N-acylglucaminen, die eine C₁₂-, C₁₄- oder einen ungesättigte C₁₈-Acylgruppe enthalten, bei mindestens 70 Gew.-% und der Anteil an N-Methyl-N-acylglucaminen, die eine Acylgruppe < C₁₂ enthalten bei weniger als 3 Gew.-%.

Insbesondere bevorzugt liegt der Anteil an N-Methyl-N-acylglucaminen, die eine C₁₂-, C₁₄- oder eine ungesättigten C₁₈-Acylgruppe enthalten, bei mindestens 80 Gew.-% und der Anteil an N-Methyl-N-acylglucaminen, die eine Acylgruppe < C₁₂ enthalten bei weniger als 2 Gew.-%.

In einer weiteren Ausführungsform liegt der Anteil an N-Methyl-N-acylglucaminen, die eine C₁₂- oder eine C₁₄-Acylgruppe enthalten, bei mindestens 90 Gew.-% und der Anteil an N-Methyl-Nacylglucaminen, die eine Acylgruppe < C₁₂ enthalten bei weniger als 2 Gew.-%.

Daneben enthalten die erfindungsgemäß als Verdicker verwendeten N-Methyl-N-acylglucamine geringe Anteile an von kurzkettigen und/oder langkettigen Fettsäuren abgeleiteten N-Methyl-N-acylglucaminen, insbesondere solchen, welche C₁-C₄-Acyl, C₆-, C₈-, C₁₀-, C₁₆-, C₁₈- und/oder C₂₀-Acylgruppen enthalten.

Im Rahmen einer weiteren bevorzugten Ausführungsform besteht die Komponente B aus einer Mischung aus N-Methyl-N-acylglucaminen, wobei mindestens 80 Gew.-% der N-Methyl-N-acylglucamine einen gesättigten oder ungesättigten C₁₆- oder C₁₈-Acylrest aufweisen. Beispielsweise können in der Mischung auch N-Methyl-N-acylglucamine vorliegen, die einen C₁₄-Acylrest aufweisen, wobei aber mindestens 80 Gew.-% der N-Methyl-N-acylglucamine einen gesättigten oder ungesättigten C₁₆- oder C₁₈-Acylrest aufweisen. Bevorzugt besteht die Komponente C aus einer Mischung aus N-Methyl-N-acylglucaminen, wobei mindestens 90 Gew.-% der N-Methyl-N-acylglucamine einen gesättigten oder ungesättigten C₁₆- oder C₁₈-Acylrest aufweisen.

### (C)

Die Fettsäuren der Komponente (C) sind bevorzugt natürliche Fettsäuren, bevorzugt mit 8 bis 22 C-Atomen, beispielsweise Octansäure, Decansäure, Laurinsäure, Myristinsäure, Palmitinsäure, Stearinsäure und Behensäure und deren Mischungen, sowie deren Alkalisalze wie Natriumstearat, Natriumpalmitat, Natriumlaurat, Natriummyristat, Natriumbehenat, Kaliumstearat, Kaliumpalmitat, Natriummyristat, Hydroxyfettsäuren, beispielsweise 12-Hydroxystearinsäure oder 16-Hydroxyhexadecanoylsäure und deren Salze. Bevorzugt sind Laurinsäure, Stearinsäure sowie deren Mischungen und Salze. Die Fettsäuren werden im Allgemeinen verwendet, um der Zusammensetzung ein rückfettendes und pflegendes Hautgefühl zu verleihen.

### (D)

Die Ölkörper als Komponente (D) können vorteilhafterweise ausgewählt werden aus den Gruppen der natürlichen und synthetische Fettkörper, vorzugsweise Triglyceride, Ester von Fettsäuren mit Alkoholen niedriger C-Zahl, z. B. mit Isopropanol, Propylenglycol oder Glycerin, oder Ester von Fettalkoholen mit Alkansäuren niedriger C-Zahl oder mit Fettsäuren oder aus der Gruppe der Alkylbenzoate, sowie natürliche oder synthetische Kohlenwasserstofföle und Silikonöle.

Besonders bevorzugt sind Triglyceridöle wie Sonneblumen- und Sojaöl; ebenso besonder bevorzugt ist Petrolatum (Vaseline).

In Betracht kommen bevorzugt Triglyceride von linearen oder verzweigten, gesättigten oder ungesättigten, gegebenenfalls hydroxylierten, C₈-C₃₀-Fettsäuren, insbesondere pflanzliche Öle, wie Sonnenblumen-, Mais-, Soja-, Reis-, Jojoba-, Babusscu-, Kürbis-, Traubenkern-, Sesam-, Walnuss-, Aprikosen-, Orangen-, Weizenkeim-, Pfirsichkern-, Makadamia-, Avocado-, Süßmandel-, Wiesenschaumkraut-, Ricinusöl, Olivenöl, Erdnussöl, Rapsöl und Kokosnussöl, sowie synthetische Triglyceridöle, z. B. das Handelsprodukt Myritol^{®} 318. Auch gehärtete Triglyceride sind erfindungsgemäß bevorzugt. Auch Öle tierischen Ursprungs, beispielweise Rindertalg, Perhydrosqualen, Lanolin können eingesetzt werden.

Weiterhin erfindungsgemäß bevorzugte Ölkörpern sind die Benzoesäureester von linearen oder verzweigten C₈₋₂₂-Alkanolen, z. B. die Handelsprodukte Finsolv^{®} SB (Isostearylbenzoat), Finsolv^{®} TN (C₁₂-C₁₅-Alkylbenzoat) und Finsolv^{®} EB (Ethylhexylbenzoat).

Eine weitere Klasse von erfindungsgemäß bevorzugten Ölkörper sind Dialkylether mit insgesamt 12 bis 36 Kohlenstoffatomen, insbesondere mit 12 bis 24 Kohlenstoffatomen, wie z. B. Di-n-octylether (Cetiol^{®} OE), Di-n-nonylether, Di-n-decylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether, Di-3-ethyldecylether, tert.-Butyl-n-octylether, iso-Pentyl-n-octylether und 2-Methylpentyl-n-octylether sowie Di-tert.-butylether und Di-isopentylether.

Ebenso in Betracht kommen verzweigte gesättigte oder ungesättigte Fettalkohole mit 6 - 30 Kohlenstoffatomen, z. B. Isostearylalkohol, sowie Guerbetalkohole.

Eine weitere Klasse von erfindungsgemäß bevorzugten Ölkörpern sind Dicarbonsäureester von linearen oder verzweigten C₂-C₁₀-Alkanolen, wie Di-n-butyladipat (Cetiol^{®} B), Di-(2-ethylhexyl)-adipat und Di-(2-ethylhexyl)-succinat sowie Diolester wie Ethylenglykoldioleat, Ethylenglykoldiisotridecanoat, Propylenglycol-di-(2-ethylhexanoat), Propylenglycoldiisostearat, Propylenglycol-dipelargonat, Butandiol-di-isostearat und Neopentylglycoldicaprylat sowie Diisotridecylacetat.

Ebenso bevorzugte Ölkörper sind symmetrische, unsymmetrische oder cyclische Ester der Kohlensäure mit Fettalkoholen, Glycerincarbonat oder Dicaprylylcarbonat (Cetiol^{®} CC).

Eine weitere Klasse von erfindungsgemäß bevorzugten Ölkörpern sind die Ester von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertig linearen oder verzweigten C₂-C₆-Alkanolen.

Eine weitere Klasse von erfindungsgemäß bevorzugten Ölkörpern sind Kohlenwasserstofföle, zum Beispiel solche mit linearen oder verzweigten, gesättigten oder ungesättigten C₇-C₄₀-Kohlenstoffketten, beispielsweise Vaseline, Dodecan, Isododecan, Cholesterol, Lanolin, synthetische Kohlenwasserstoffe wie Polyolefine, insbesondere Polyisobuten, hydriertes Polyisobuten, Polydecan, sowie Hexadecan, Isohexadecan, Paraffinöle, Isoparaffinöle, z. B. die Handelsprodukte der Permethyl^{®}-Serie, Squalan, Squalen, und alicyclische Kohlenwasserstoffe, z. B. das Handelsprodukt 1,3-Di-(2-ethyl-hexyl)-cyclohexan (Cetiol^{®} S), Ozokerit und Ceresin.

### (E)

Im Rahmen einer bevorzugten Ausführungsform umfasst die erfindungsgemäße Zusammensetzung mindestens ein Acylisethionat der Formel (II) als Komponente (E):

R-CO-O-CHR¹-CHR²-SO₃X (II)

worin
- R: den Alkylrest einer C₈-C₁₈-Fettsäure bedeutet,
- R¹ und R²: unabhängig voneinander H oder CH₃, vorzugsweise H, bedeuten und
- X: ein Kation, vorzugsweise ein Alkalimetallkation, insbesondere Na, ist.

Hierunter fallen Acylisethionate und Methyl-acylisethionate mit einem C₈-C₁₈ Acylrest und deren Mischungen, bevorzugt deren Natriumsalze. Besonders bevorzugt sind Natriumcocoylisethionate und Natriumlauroylisethionat.

Zusammensetzungen, die auch die Komponente (E) enthalten, zeigen eine besonders gute Stabilität.

### (F)

Im Rahmen einer bevorzugten Ausführungsform umfasst die erfindungsgemäße Zusammensetzung mindestens ein anionisches, sulfoniertes Tensid als Komponente (F).

In einer bevorzugten Ausführungsform ist die Komponente F ausgewählt aus einer oder mehreren Verbindung(en) der allgemeinen Formel (III)

R¹SO₃⁻ M⁺ (III)

wobei
R¹ für Alkyl, Cycloalkyl, Aralkyl, Aryl, Alkoxy, Alkoxyalkyl und Heterocyclyl steht und
M⁺ ein Alkalimetall-, Erdalkalimetall- oder ein substituiertes oder nicht substituiertes Ammoniumion ist, oder
der allgemeinen Formel (IV)

R¹SO₄⁻ M⁺ (IV)

wobei
R¹ für Alkyl, Cycloalkyl, Aralkyl, Aryl, Alkoxy, Alkoxyalkyl und Heterocyclyl steht und
M⁺ ein Alkalimetall-, Erdalkalimetall- oder ein substituiertes oder nicht substituiertes Ammoniumion ist.

"Alkyl" bedeutet eine gesättigte aliphatische Kohlenwasserstoffgruppe, die geradkettig oder verzweigt sein kann und von 1 bis 20 Kohlenstoffatome in der Kette haben kann. Bevorzugte Alkylgruppen können geradkettig oder verzweigt sein und von 1 bis zu 10 Kohlenstoffatome in der Kette aufweisen. Verzweigt bedeutet, dass eine Nieder-Alkylgruppe, wie Methyl, Ethyl oder Propyl, an eine lineare Alkylkette angebracht ist. Bei Alkyl handelt es sich beispielsweise um Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, 1-Heptyl, 1-Octyl, 1-Nonyl, 1-Decyl, 1-Undecyl, 1-Dodecyl, 1-Tetradecyl, 1-Hexadecyl und 1-Octadecyl.

"Cycloalkyl" bedeutet einen aliphatischen Ring, der von 3 bis 10 Kohlenstoffatome in dem Ring hat. Bevorzugte Cycloalkylgruppen haben von 4 bis 7 Kohlenstoffatome in dem Ring.

"Aryl" bedeutet Phenyl oder Naphthyl.

"Aralkyl" bedeutet eine Alkylgruppe, die mit einem Arylrest substituiert ist.

"Substituiertes Aralkyl" und "substituiertes Aryl" bedeuten, dass die Arylgruppe oder die Arylgruppe der Aralkylgruppe mit einem oder mehreren Substituenten ausgewählt aus Alkyl, Alkoxy, Nitro, Carboalkoxy, Cyano, Halo, Alkylmercaptyl, Trihaloalkyl oder Carboxyalkyl substituiert ist.

"Alkoxy" bedeutet eine Alkyl-O-Gruppe, in der "Alkyl" die vorstehend beschriebene Bedeutung hat. Nieder-Alkoxy-Gruppen sind bevorzugt. Beispiele sind Methoxy, Ethoxy, n-Propoxy, i-Propoxy und n-Butoxy.

"Nieder-Alkyl" bedeutet eine Alkylgruppe, die 1 bis 7 Kohlenstoffatome aufweist. "Alkoxyalkyl" bedeutet eine Alkylgruppe wie vorstehend beschrieben, die mit einer Alkoxygruppe, wie vorstehend beschrieben, substituiert ist. Somit kann unter dem Begriff Alkoxyalkyl ein Polyether verstanden werden.

"Heterocyclyl" bedeutet eine 4 bis 10-gliedrige Ringstruktur, in der ein oder mehrere Ringatome von Kohlenstoff verschieden sind, beispielsweise N, O oder S sind. Heterocyclyl kann aromatisch oder nicht-aromatisch sein, d. h. es kann gesättigt, teilweise oder ganz ungesättigt sein.

Im Rahmen einer bevorzugten Ausführungsform ist das anionische Tensid der Komponente A ein Alkylsulfat oder ein Alkylethersulfat. Besonders bevorzugt sind Natriumlaurylsulfat, Natriumlaurethsulfat (1 oder 2 EO-Einheiten) bzw. deren Mischungen.

In einer weiteren bevorzugten Ausführungsform enthält die erfindungsgemäße Zusammensetzung keine Komponente (F).

### (G)

Im Rahmen einer bevorzugten Ausführungsform umfasst die erfindungsgemäße Zusammensetzung mindestens ein Alkylbetain und/oder mindestens ein Alkylamidobetain als Komponente (G).

Beispiele für geeignete Alkylbetaine stellen die Carboxyalkylierungsprodukte von sekundären und insbesondere tertiären Aminen der Formel (V) dar 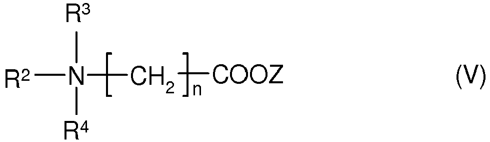 in der
- R²: für Alkyl- und/oder Alkenylreste mit 6 bis 22 Kohlenstoffatomen,
- R³: für Wasserstoff oder Alkylreste mit 1 bis 4 Kohlenstoffatomen, R⁴ für Wasserstoff oder Alkylreste mit 1 bis 4 Kohlenstoffatomen,
- n: für Zahlen von 1 bis 6 und
- Z: für ein Alkali- und/oder Erdalkalimetall oder Ammonium steht.

Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, C₁₂/₁₄-Kokosalkyldimethylamin, Myristyldimethylamin, Cetyldimethylamin, Stearyldimethylamin, Stearylethylmethylamin, Oleyldimethylamin, C₁₆-C₁₈-Talgalkyldimethylamin sowie deren technische Gemische.

Beispiele für geeignete Alkylamidobetaine stellen Carboxyalkylierungsprodukte von Amidoaminen da. Insbesondere geeignet sind Amidopropylbetaine der Formel (VI), 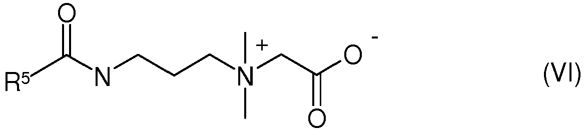 worin R⁵ eine lineare oder verzweigte gesättigte C₇-C₂₁ Alkylgruppe oder eine lineare oder verzweigte ein- oder mehrfach ungesättigte C₇-C₂₁ Alkenylgruppe ist.

Bevorzugten Betaintenside sind Amidopropylbetaine wie Cocoamidopropylbetain (R⁵CO ist der Fettsäurerest des Cocosöls, Kettenlänge C₈-C₁₈) und Alkylbetaine wie Coco-Betain (R² ist der Alkylrest des Cocosöls, Kettenlänge C₈-C₁₈) oder Laurylbetain (R² ist ein Alkylrest der Kettenlänge C₁₂ und C₁₄).

### (H)

In Rahmen einer bevorzugten Ausführungsform enthält die erfindungsgemäße Zusammensetzung ein oder mehrere Additive, bevorzugt aus der Gruppe bestehend aus Konservierungsmitteln, Duftstoffen, Farbstoffen, weiteren Tensiden, die nicht unter die Definition der Komponenten A-G fallen, Lösungsmitteln, kationische Polymeren, Filmbildnern, Verdickungs- und Gelierungsmitteln, Überfettungsmitteln, antimikrobiellen und biogenen Wirkstoffen, feuchtigkeitsspendenden Mitteln, Stabilisatoren, Säuren, Laugen, Wirkverstärkern und Mischungen daraus, bevorzugt in Mengen von 0,1 bis 10,0 Gew.-%, besonders bevorzugt von 3,0 bis 15,0 Gew.-% und insbesondere von 5,0 bis 10,0 Gew.-%.

Als Konservierungsmittel eignen sich alle im betreffenden Annex der europäischen Kosmetikgesetzgebung gelisteten Konservierungsmittel, beispielsweise Phenoxyethanol, Benzylalkohol, Parabene, Benzoesäure und Sorbinsäure, besonders gut geeignet ist beispielsweise 1,3-Bis(hydroxymethyl)-5,5-dimethylimidazolidine-2,4-dione (Nipaguard^{®} DMDMH), Pirocton Olamine, Methylisothiazolinon oder Mischungen daraus, bevorzugt Pirocton Olamin und/oder Methylisothiazolinon.

Als Duft- bzw. Parfümstoffe oder Öle können einzelne Riechstoffverbindungen, z. B. die synthetischen Produkte vom Typ der Ester, Ether, Aldehyde, Ketone, Alkohole und Kohlenwasserstoffe verwendet werden. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethyl-methylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z. B. die lonone, alpha-Isomethylionon und Methyl-cedrylketon, zu den Alkoholen Anethol, Citronellol, Eugenol, Geranion, Linalol, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z. B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Als Farbstoffe eignen sich im Prinzip alle Farbstoffe, die für den Kosmetikgebrauch zugelassen sind, diese sind in den entsprechenden Anhängen der europäischen Kosmetikgesetzgebung gelistet sind.

Weitere Tenside, die nicht unter die Definition von (A)-(G) fallen können im Prinzip alle anionischen, kationischen oder amphoteren Tenside sein, die kosmetikgeeignet sind. Bevorzugt sind:
Ethoxylierte und propoxylierte Fettalkohole, ethoxylierte und propoxylierte Triglyceride wie PEG-40 hydrogenated Castor Oil oder Fettsäureester, Ethercarboxylate, Alkylpolyglucoside, Olefinsulfonate, sec. Alkylsulfonate und Tau rate.

Unter einem Lösungsmittel im Rahmen der vorliegenden Erfindung versteht man vorzugsweise protische Lösungsmittel wie Wasser, C₁-C₈-Alkohole, insbesondere C₁-C₆-Alkohole, Ethylenglykol, Diethylenglykol, Triethylenglykol oder Mischungen davon, wobei insbesondere Wasser und/oder Ethanol oder Wasser und/oder Methanol bevorzugt sind. Von den C₁-C₆-Alkoholen sind Methanol, Ethanol, Isopropanol, n-Butanol oder sek.-Butanol bevorzugt.

Als kationische Polymere eignen sich die unter der INCI-Bezeichnung "Polyquaternium" bekannten, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, sowie Polyquaternium 37&mineral oil&PPG trideceth (Salcare SC95), PVP-dimethylaminoethylmethacrylat-Copolymer, Guarhydroxypropyltriammoniumchloride, sowie Calciumalginat und Ammoniumalginat. Des Weiteren können eingesetzt werden kationische Cellulosederivate; kationische Stärke; Copolymere von Diallylammoniumsalzen und Acrylamiden; quaternierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z. B. Amidomethicone; Copolymere der Adipinsäure und Dimethylaminohydroxypropyldiethylentriamin; Polyaminopolyamid und kationische Chitinderivate, wie beispielsweise Chitosan.

Des Weiteren können die erfindungsgemäßen Zusammensetzungen Filmbildner enthalten, die je nach Anwendungszweck ausgewählt sind aus Salzen der Phenylbenzimidazolsulfonsäure, wasserlöslichen Polyurethanen, beispielsweise C₁₀-Polycarbamylpolyglycerylester, Polyvinylalkohol, Polyvinylpyrrolidoncopolymeren wie PVP/Hexandecene oder PVP/Eicosene Copolymer, beispielsweise Vinylpyrrolidon/Vinylacetatcopolymer, wasserlöslichen Acrylsäurepolymeren/Copolymeren bzw. deren Estern oder Salzen, beispielsweise Partialestercopolymere der Acryl/Methacrylsäure und Polyethylenglykolethern von Fettalkoholen, wie Acrylat/Steareth-20-Methacrylat Copolymer, wasserlöslicher Cellulose, beispielsweise Hydroxymethylcellulose, Hydroxyethylcellulose, Hydroxypropylcellulose, wasserlöslichen Quaterniums, Polyquaterniums, Carboxyvinyl-Polymeren, wie Carbomere und deren Salze, Polysacchariden, beispielsweise Polydextrose und Glucan, Vinylacetat/Crotonat, beispielsweise unter dem Handelsnamen Aristoflex^{®} A 60 (Clariant) erhältlich, sowie polymeren Aminoxiden, beispielsweise unter den Handelsnamen Diaformer Z-711, 712, 731, 751 erhältliche Vertreter.

Die gewünschte Viskosität der Zusammensetzungen kann durch Zugabe von Verdickern und Gelierungsmittel eingestellt (erhöht oder erniedrigt) werden. In Betracht kommen vorzugsweise Celluloseether und andere Cellulosederivate (z. B. Carboxymethylcellulose, Hydroxyethylcellulose), Gelatine, Stärke und Stärkederivate wie Hydroxypropyl Stärke Phosphate oder Natrium Hydroxypropyl Stärke Phosphate, Natriumalginate, Fettsäurepolyethylenglykolester, Agar-Agar, Traganth oder Dextrinderivate, insbesondere Dextrinester. Des Weiteren eignen sich Fettsäureamide; Fettsäurealkanolamide; Dibenzalsorbit und alkohollösliche Polyamide und Polyacrylamide oder Mischungen solcher. Weiterhin können vernetzte und unvernetzte Polyacrylate wie Carbomer, Natriumpolyacrylate oder sulfonsäurehaltige Polymere wie Ammoniumacryloyldimethyltaurate/VP-Copolymer Verwendung finden.

Als Überfettungsmittel können vorzugsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide, sowie ethoxylierte Triglyceride wie PEG-7 Glyceryl Cocoat oder Mischungen aus Glyceryloleat mit Alkylpolyglucosiden Verwendung finden.

An antimikrobiellen Wirkstoffen kommen Cetyltrimethylammoniumchlorid, Cetylpyridiniumchlorid, Benzethoniumchlorid, Diisobutylethoxyethyldimethylbenzylammoniumchlorid, Natrium N-Laurylsarcosinat, Natrium-N-Palmethylsarcosinat, Lauroylsarcosin, N-Myristoylglycin, Kalium-N-Laurylsarcosin, Trimethylammoniumchlorid, Natrium-aluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Farnesol, Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Selendisulfid und Octopirox, lodopropynylbutylcarbamat, Methylchloroisothiazolinon, Methylisothiazolinon, Methyldibromo Glutaronitril, AgCl, Chloroxylenol, Na-Salz von Diethylhexylsulfosuccinat, Natriumbenzoat, sowie Phenoxyethanol, Benzylalkohol, Phenoxyisopropanol, Parabene, bevorzugt Butyl-, Ethyl-, Methyl- und Propylparaben, sowie deren Na-Salze, Pentandiol, 1,2-Octandiol, 2-Bromo-2-Nitropropan-1,3-diol, Ethylhexylglycerin, Benzylalkohol, Sorbinsäure, Benzoesäure, Milchsäure, Imidazolidinylharnstoff, Diazolidinylharnstoff, Dimethyloldimethylhydantoin (DMDMH), Na-Salz von Hydroxymethylglycinat, Hydroxyethylglycin der Sorbinsäure und Kombinationen dieser Wirksubstanzen zum Einsatz.

Die erfindungsgemäßen Zusammensetzungen können des Weiteren biogene Wirkstoffe ausgewählt aus Pflanzenextrakten, wie beispielsweise Aloe Vera, sowie Lokalanästhetika, Antibiotika, Antiphlogistika, Antiallergica, Corticosteroide, Sebostatika, Bisabolol®, Allantoin®, Phytantriol®, Proteine, Vitamine ausgewählt aus Niacin, Biotin, Vitamin B2, Vitamin B3, Vitamin B6, Vitamin B3 Derivaten (Salzen, Säuren, Estern, Amiden, Alkoholen), Vitamin C und Vitamin C Derivaten (Salzen, Säuren, Estern, Amiden, Alkoholen), bevorzugt als Natriumsalz des Monophosphorsäureesters der Ascorbinsäure oder als Magnesiumsalz des Phosphorsäureesters der Ascorbinsäure, Tocopherol und Tocopherolacetat, sowie Vitamin E und/oder dessen Derivate enthalten.

Als feuchtigkeitsspendende Substanz stehen beispielsweise Isopropylpalmitat, Glycerin, Diglycerin und/oder Sorbitol zu Verfügung. Besonders bevorzugt ist Glycerin.

Als Säuren oder Laugen zur pH-Wert Einstellung werden vorzugsweise Mineralsäuren, insbesondere HCl, anorganische Basen, insbesondere NaOH oder KOH, oder organische Säuren, insbesondere Milchsäure, verwendet.

Als Wirkverstärker kann bevorzugt Sorbitan Caprylat verwendet werden.

Im Rahmen einer bevorzugten Ausführungsform ist die Zusammensetzung frei von Alkylsulfaten und/oder Alkylethersulfaten. Dabei bedeutet frei, dass die Zusammensetzung weniger als 3 Gew.-%, bezogen auf die Gesamtmenge der Zusammensetzung, bevorzugt weniger als 0,5 Gew.-%, und insbesondere keine Alkylsulfate und/oder Alkylethersulfaten beinhaltet.

Im Rahmen einer bevorzugten Ausführungsform handelt es sich bei der erfindungsgemäßen Zusammensetzung um eine kosmetische, dermatologische oder pharmazeutische Zusammensetzung.

Ein weiterer Gegenstand der Erfindung ist die Verwendung der erfindungsgemäßen Zusammensetzung als Shampoo, Gesichtsreiniger, Flüssigreiniger oder Duschbad.

Ein weiterer Gegenstand der Erfindung ist die Verwendung der erfindungsgemäßen Zusammensetzung zur Behandlung oder Pflege der Haut.

Ein weiterer Gegenstand der Erfindung ist die Verwendung der erfindungsgemäßen Zusammensetzung zur Behandlung oder Pflege der Haare.

Die Erfindung wird durch die nachstehenden Beispiele näher erläutert.

### Herstellbeispiel H1 und H2

Die im Folgenden beschriebene N-Acyl-N-methyl-glucamine wurden nach EP 0 550 637 aus den korrespondierenden Fettsäuremethylestern und N-Methylglucamin in Gegenwart von 1,2 Propylenglykol als Lösemittel hergestellt und als Feststoff bestehend aus Aktivsubstanz und 1,2 Propylenglykol erhalten.

**Tabelle 1**

| Herstell-beispiel | Methylester | Aktivsubstanz (%) | 1,2-Propylenglykol (%) | Schmelzpunkt (°C) |
|---|---|---|---|---|
| H1 | C12/18 | 88 | 12 | 80 |
| H2 | C 16/18 | 80 | 20 | 68 |

C12/C18 bedeutet, dass der Methylester aus einem Gemisch aus Laurinsäuremethylester (C₁₂-Acylrest), Myristinsäuremethylester (C₁₄-Acylrest), Palmitinsäuremethylester (C₁₆-Acylrest), Stearinsäuremethylester (C₁₈-Acylrest) und Ölsäuremethylester (C₁₈-Acylrest) besteht (Verhältnis 64 : 21 : 2 : 3 : 10). C16/18 bedeutet, dass der Methylester aus einem Gemisch aus Palmitinsäuremethylester (C₁₆-Acylrest) und Stearinsäuremethylester (C₁₈-Acylrest) besteht (Verhältnis 30 : 70).

Die Viskositäten werden mit einem Brookfield-Viskosimeter Model DV II, den Spindeln aus dem Spindelset RV bei 20 Umdrehungen / Minute und 20 °C gemessen. Es werden die Spindeln 1 bis 7 aus dem Spindelset RV verwendet. Unter diesen Messbedingungen wird Spindel 1 für Viskositäten von maximal 500 mPa·s, Spindel 2 für Viskositäten von maximal 1 000 mPa·s, Spindel 3 für Viskositäten von maximal 5 000 mPa·s, Spindel 4 für Viskositäten von maximal 10 000 mPa·s, Spindel 5 für Viskositäten von maximal 20 000 mPa·s, Spindel 6 für Viskositäten von maximal 50 000 mPa·s und Spindel 7 für Viskositäten von maximal 200 000 mPa·s gewählt.

In der folgenden Testformulierung wurden ölhaltige Cremeduschbäder mit und ohne Zusatz von N-Acyl-Methylglucamin nach Herstellbeispiel H1 und H2 im Vergleich zu Formulierungen ohne N-Acyl-Methylglucamin getestet. Die jeweiligen Einsatzmengen beziehen sich auf den Aktivgehalt der Komponenten, d. h. 2,0 % des Herstellbeispiels H1 entsprechen 2,27 % tatsächliche Einsatzmenge des Reaktionsprodukts.

Dabei ist zu erkennen, dass sich dann stabile Emulsionen ergeben, wenn neben Ölkörpern und einer Fettsäure gleichzeitig Aminosäuretensid (Na-Cocoyl Glycinat) und N-Acyl-Methylglucamin anwesend sind (erfindungsgemäße Formulierungen Beispiele 1-4).

| Formulierung Beispiel Komponente (%) | V1 | V2 | V3 | V4 | V5 | V6 | 1 | 2 | 3 | 4 | V7 | V8 | V9 | V10 |
|---|---|---|---|---|---|---|---|---|---|---|---|---|---|---|
| Wasser | ad 100 | ad 100 | ad 100 | ad 100 | ad 100 | ad 100 | ad 100 | ad 100 | ad 100 | ad 100 | ad 100 | ad 100 | ad 100 | ad 100 |
| Sodium Lauryl ethersulfat (2 EO) | 5,0 | 6,0 | 5,0 | 6,0 | 5,0 | 6,0 | 5,0 | 6,0 | 5,0 | 6,0 | 7,0 | 8,0 | 7,0 | 8,0 |
| Sodium Cocoyl Glycinat | 2,0 | 2,0 | 0,0 | 0,0 | 0,0 | 0,0 | 2,0 | 2,0 | 2,0 | 2,0 | 0,0 | 0,0 | 0,0 | 0,0 |
| Herstellbeispiel H1 | 0,0 | 0,0 | 2,0 | 2,0 | 0,0 | 0,0 | 2,0 | 2,0 | 0,0 | 0,0 | 2,0 | 2,0 | 0,0 | 0,0 |
| Herstellbeispiel H2 | 0,0 | 0,0 | 0,0 | 0,0 | 2,0 | 2,0 | 0,0 | 0,0 | 2,0 | 2,0 | 0,0 | 0,0 | 2,0 | 2,0 |
| Laurinsäure | 2,0 | 2,0 | 2,0 | 2,0 | 2,0 | 2,0 | 2,0 | 2,0 | 2,0 | 2,0 | 2,0 | 2,0 | 2,0 | 2,0 |
| Sonnenblumenöl | 1,5 | 1,0 | 1,5 | 1,0 | 1,5 | 1,0 | 1,5 | 1,0 | 1,5 | 1,0 | 1,5 | 1,0 | 1,5 | 1,0 |
| Parfum Wasserlilie | 1,0 | 1,0 | 1,0 | 1,0 | 1,0 | 1,0 | 1,0 | 1,0 | 1,0 | 1,0 | 1,0 | 1,0 | 1,0 | 1,0 |
| Sodium Cocoyl Isethionat | 1,2 | 1,2 | 1,2 | 1,2 | 1,2 | 1,2 | 1,2 | 1,2 | 1,2 | 1,2 | 1,2 | 1,2 | 1,2 | 1,2 |
| Glycerin | 1,4 | 1,0 | 1,4 | 1,0 | 1,4 | 1,0 | 1,4 | 1,0 | 1,4 | 1,0 | 1,4 | 1,0 | 1,4 | 1,0 |
| Sodium Chlorid | 0,5 | 0,5 | 0,5 | 0,5 | 0,5 | 0,5 | 0,5 | 0,5 | 0,5 | 0,5 | 0,5 | 0,5 | 0,5 | 0,5 |
| Stearinsäure | 1,0 | 1,0 | 1,0 | 1,0 | 1,0 | 1,0 | 1,0 | 1,0 | 1,0 | 1,0 | 1,0 | 1,0 | 1,0 | 1,0 |
| Cocamidopropyl Betain | 0,5 | 0,2 | 0,5 | 0,2 | 0,5 | 0,2 | 0,5 | 0,2 | 0,5 | 0,2 | 0,5 | 0,2 | 0,5 | 0,2 |

| Zitronensäure bis | pH 6,2 | pH 6,2 | pH 6,2 | pH 6,2 | pH 6,2 | pH 6,2 | pH 6,2 | pH 6,2 | pH 6,2 | pH 6,2 | pH 6,2 | pH 6,2 | pH 6,2 | pH 6,2 |
|---|---|---|---|---|---|---|---|---|---|---|---|---|---|---|
| DMDM Hydantoin | 0,2 | 0,2 | 0,2 | 0,2 | 0,2 | 0,2 | 0,2 | 0,2 | 0,2 | 0,2 | 0,2 | 0,2 | 0,2 | 0,2 |
| Stabilität nach 1 Tag | nein | nein | nein | nein | nein | nein | ja | ja | ja | ja | nein | nein | nein | nein |
| Stabilität nach 1 Woche | nein | nein | nein | nein | nein | nein | ja | ja | ja | ja | nein | nein | nein | nein |
| Viskosität 4 °C (mPas) | - | - | - | - | - | - | 258 | 194 | 228 | 218 | - | - | - | - |
| Viskosität 20 °C (mPas) | - | - | - | - | - | - | 296 | 140 | 166 | 102 | - | - | - | - |
| Viskosität 40 °C (mPas) | - | - | - | - | - | - | 282 | 110 | 154 | 78 | - | - | - | - |

Die durchgeführten Versuche zeigen auch, dass man dann eine stabile Emulsion erhält, wenn gleichzeitig Glycinattensid und Glucamidtensid in der Zusammensetzung enthalten sind. Tauscht man dagegen das Glycinattensid in Gegenwart von Glucamiden gegen Natriumlaurylethersulfat aus, sind keine stabilen Emulsionen zu sehen.

In den weiteren Beispielen wurde ein ölhaltiges Duschbad mit Hydroxypropylstärkephoshat verdickt, um gutes rheologisches Verhalten bei der Applikation zu erhalten.

| Formulierung Beispiel Komponente (%) | 5 | V11 |
|---|---|---|
| Wasser | add 100 | add 100 |
| Na-Laureth Sulfat (2 EO) | 3 | 3 |
| Na-Cocoyl Glycinat | 2 | 2 |
| Hydroxypropyl stärkephosphat | 2 | 2 |
| Herstellbeispiel H 1 | 2 | 0 |
| Laurinsäure | 1.6 | 1.6 |
| Sonnenblumenöl | 1.2 | 1.2 |
| Parfum Wasserlilie | 1 | 1 |
| Na- Cocoyl sethionat | 1 | 1 |
| Glycerin | 1 | 1 |
| NaCl | 1 | 1 |
| Stearinsäure | 2 | 2 |
| Cocamidopropyl Betain | 3 | 3 |
| Zitronensäure | pH 6.2 | pH 6.2 |
| DMDM Hydantoin | 0.2 | 0.2 |
| Viskosität bei 4 °C (mPas) | 3000 | 3020 |
| Viskosität bei 20 °C (mPas) | 4165 | 2410 |
| Viskosität bei 40 °C (mPas) | 4650 | 2660 |

Wie man aus Vergleichsbeispiel 11 und Beispiel 5 sieht, führt die Zugabe der Komponente B (Herstellbeispiel 1) bei konstanter Verdickerkonzentration (Hydroxypropylstärkephosphat) zu einer höheren Viskosität bei 20 °C und 40 °C. Dies ist ein erwünschter Effekt, weil man einerseits zur Erreichung der Zielviskosität weniger Polymer benötigt, andererseits durch das bessere Viskositätsprofil bei 40 °C lagerstabilere Emulsionen erhält.

## Patentansprüche

1. Zusammensetzung enthaltend:
(A) mindestens ein N-Acyl-aminosäuretensid als Komponente A, wobei Komponente A aus mindestens einer C₈-C₂₂-acylierten Aminosäure besteht, und ausgewählt ist aus der Gruppe bestehend aus Acylglycinat, Acylaspartat, Acylglutamat, Acylsarkosinat, deren Salzen und Mischungen davon,
(B) mindestens ein N-Methyl-N-acylglucamin als Komponente B,
(C) mindestens eine C₈-C₂₂ Fettsäure oder ein Fettsäuresalz als Komponente C,
(D) mindestens einen Ölkörper als Komponente D,
(E) optional mindestens ein Acylisethionat als Komponente E,
(F) optional mindestens ein anionisches, sulfoniertes Tensid als Komponente F,
(G) optional mindestens ein Betaintensid als Komponente G,
(H) optional mindestens ein Additiv als Komponente H und
(I) Wasser.

2. Zusammensetzung nach Anspruch 1 enthaltend:
(A) mindestens ein N-Acyl-aminosäuretensid als Komponente A, wobei Komponente A aus mindestens einer C₈-C₂₂-acylierten Aminosäure besteht, und ausgewählt ist aus der Gruppe bestehend aus Acylglycinat, Acylaspartat, Acylglutamat, Acylsarkosinat, deren Salzen und Mischungen davon,
(B) mindestens ein N-Methyl-N-acylglucamin als Komponente B,
(C) mindestens eine C₁₂-C₂₂ Fettsäure oder Fettsäuresalz als Komponente C,
(D) mindestens einen Ölkorper als Komponente D,
(E) mindestens ein Acylisethionat als Komponente E,
(F) optional mindestens ein anionisches, sulfoniertes Tensid als Komponente F,
(G) optional mindestens ein Betaintensid als Komponente G,
(H) optional mindestens ein Additiv als Komponente H und
(I) Wasser.

3. Zusammensetzung nach Anspruch 1, bestehend aus:
| | | | |
|---|---|---|---|
| (A) | 1 | -10 | Gew.-% der Komponente A, |
| (B) | 0,5 | - 10 | Gew.-% der Komponente B, |
| (C) | 0,5 | - 5,0 | Gew.-% der Komponente C, |
| (D) | 0,5 | - 8,0 | Gew.-% der Komponente D, |
| (E) | 0 | - 5,0 | Gew-% der Komponente E, |
| (F) | 0 | - 10 | Gew.-% der Komponente F, |
| (G) | 0 | - 5 | Gew.-% der Komponente G, |
| (H) | 0 | - 15 | Gew.-% eines oder mehrerer Additive H und |
| (I) | 74 | - 98 | Gew-% Wasser |
wobei die Summe der Komponenten A bis I 100 Gew.-% ergibt.

4. Zusammensetzung nach Anspruch 2, bestehend aus:
| | | | |
|---|---|---|---|
| (A) | 1 | - 10 | Gew.-% der Komponente A, |
| (B) | 0,5 | - 10 | Gew.-% der Komponente B, |
| (C) | 0,5 | - 5,0 | Gew.-% der Komponente C, |
| (D) | 0,5 | - 8,0 | Gew.-% der Komponente D, |
| (E) | 0,5 | bis 5,0 | Gew-% der Komponente E, |
| (F) | 0 | - 10 | Gew.-% der Komponente F, |
| (G) | 0 | - 5 | Gew.-% der Komponente G, |
| (H) | 0 | - 15 | Gew.-% eines oder mehrerer Additive H und |
| (I) | 74 | - 97,5 | Gew-% Wasser |
wobei die Summe der Komponenten A bis I 100 Gew.-% ergibt.

5. Zusammensetzung nach einem der Ansprüche 1 bis 4, bestehend aus:
| | | | |
|---|---|---|---|
| (A) | 1 | - 6,0 | Gew.-% der Komponente A, |
| (B) | 1 | - 5,0 | Gew.-% der Komponente B, |
| (C) | 1 | - 4,0 | Gew.-% der Komponente C, |
| (D) | 1 | - 5,0 | Gew.-% der Komponente D, |
| (E) | 0,5 | bis 2,0 | Gew-% der Komponente E, |
| (F) | 1 | - 5 | Gew.-% der Komponente F, |
| (G) | 0,5 | - 3 | Gew.-% der Komponente G, |
| (H) | 5 | - 10 | Gew.-% eines oder mehrerer Additive H |
| (I) | 40 | - 89 | Gew-% Wasser |
wobei die Summe der Komponenten A bis I 100 Gew.-% ergibt.

6. Zusammensetzung nach einem der Ansprüche 1 bis 5, **dadurch gekennzeichnet, dass** die Komponente A ausgewählt ist aus der Gruppe bestehend aus Acylglycinat, dessen Salzen und Mischungen davon.

7. Zusammensetzung nach einem der Ansprüche 1 bis 6, **dadurch gekennzeichnet, dass** die Komponente B aus einem oder mehreren C₈-C₂₂ N-Methyl-N-acylglucaminen besteht.

8. Zusammensetzung nach Anspruch 7, **dadurch gekennzeichnet, dass** die Komponente B aus mindestens einem C₁₂-C₁₈ N-Methyl-N-acylglucamin besteht.

9. Zusammensetzung nach einem der Ansprüche 1 bis 8 **dadurch gekennzeichnet, dass** die Komponente C aus Laurinsäure, Palmitinsäure, Stearinsäure, deren Salzen oder Mischungen daraus besteht.

10. Zusammensetzung nach einem der Ansprüche 1 bis 9, **dadurch gekennzeichnet, dass** die Komponente D aus einem oder einer Mischung von Ölkörpern aus der Gruppe der Triglyceridöle, der Esteröle, der Kohlenwasserstofföle und Silikonöle besteht.

11. Zusammensetzung nach Anspruch 10, **dadurch gekennzeichnet, dass** die Komponente D aus einem oder einer Mischung von Ölkörpern aus der Gruppe der Triglyceridöle, besteht.

12. Zusammensetzung nach einem der Ansprüche 1 bis 11, **dadurch gekennzeichnet, dass** die Komponente E enthalten ist und aus Natriumlauroylisethionat, Natriumcocoylisethionat oder deren Mischung besteht.

13. Zusammensetzung nach einem der Ansprüche 1 bis 12, **dadurch gekennzeichnet, dass** die Komponente F enthalten ist und aus Natriumlaurylethersulfat, Natriummyrethsulfat, Natriumlaurylsulfat, Natriumcocoylsulfat, oder Mischungen davon besteht.

14. Zusammensetzung nach einem der Ansprüche 1 bis 12, **dadurch gekennzeichnet, dass** die Zusammensetzung frei von der Komponente F ist.

15. Zusammensetzung nach einem der Ansprüche 1 bis 14, **dadurch gekennzeichnet, dass** die Komponente G enthalten ist und aus Cocamidopropylbetain, Lauramidopropylbetain, Coco-Betain oder Mischungen davon besteht.

16. Zusammensetzung nach einem der Ansprüche 1 bis 15, **dadurch gekennzeichnet, dass** ein oder mehrere Additive (H) enthalten sind, gewählt aus der Gruppe bestehend aus Konservierungsmitteln, Duftstoffen, Farbstoffen, weiteren Tensiden, die nicht unter die Definition der Komponenten A-G fallen, kationischen Polymeren, Filmbildnern, Verdickungs- und Gelierungsmitteln, Überfettungsmitteln, antimikrobiellen und biogenen Wirkstoffen, feuchtigkeitsspendenden Mitteln, Stabilisatoren, Säuren, Laugen und Wirkverstärkern.

17. Zusammensetzung nach einem der Ansprüche 1 bis 16, **dadurch gekennzeichnet, dass** die Summe der Komponenten A bis H von 10 bis 30 Gew.-% beträgt.

18. Zusammensetzung nach einem der Ansprüche 1 bis 17, **dadurch gekennzeichnet, dass** es sich bei der Zusammensetzung um eine kosmetische, dermatologische oder pharmazeutische Zusammensetzung handelt.

19. Verwendung einer Zusammensetzung nach einem der Ansprüche 1 bis 18 als Shampoo, Gesichtsreiniger, Duschbad oder Duschcreme.

20. Verwendung der Zusammensetzung nach einem der Ansprüche 1 bis 18 zur Behandlung oder Pflege der Haut, der Haare oder von Haut und Haaren.

## Claims

1. Composition comprising:
(A) at least one N-acyl-amino acid surfactant as component A, wherein component A consists of at least one C₈-C₂₂-acylated amino acid and is selected from the group consisting of acyl glycinate, acyl aspartate, acyl glutamate, acyl sarcosinate, their salts and mixtures thereof,
(B) at least one N-methyl-N-acylglucamine as component B,
(C) at least one C₈-C₂₂-fatty acid or a fatty acid salt as component C,
(D) at least one oily substance as component D,
(E) optionally at least one acyl isethionate as component E,
(F) optionally at least one anionic, sulfonated surfactant as component F,
(G) optionally at least one betaine surfactant as component G,
(H) optionally at least one additive as component H, and
(I) water.

2. Composition according to Claim 1, comprising:
(A) at least one N-acyl-amino acid surfactant as component A, wherein component A consists of at least one C₈-C₂₂-acylated amino acid and is selected from the group consisting of acyl glycinate, acyl aspartate, acyl glutamate, acyl sarcosinate, their salts and mixtures thereof,
(B) at least one N-methyl-N-acylglucamine as component B,
(C) at least one C₁₂-C₂₂-fatty acid or fatty acid salt as component C,
(D) at least one oily substance as component D,
(E) at least one acyl isethionate as component E,
(F) optionally at least one anionic, sulfonated surfactant as component F,
(G) optionally at least one betaine surfactant as component G,
(H) optionally at least one additive as component H, and
(I) water.

3. Composition according to Claim 1, consisting of:
| | |
|---|---|
| (A) | from 1 to 10% by weight of component A, |
| (B) | from 0.5 to 10% by weight of component B, |
| (C) | from 0.5 to 5.0% by weight of component C, |
| (D) | from 0.5 to 8.0% by weight of component D, |
| (E) | from 0 to 5.0% by weight of component E, |
| (F) | from 0 to 10% by weight of component F, |
| (G) | from 0 to 5% by weight of component G, |
| (H) | from 0 to 15% by weight of one or more additives H, and |
| (I) | from 74 to 98% by weight water, |
wherein the sum of components A to I is 100% by weight.

4. Composition according to Claim 2, consisting of:
| | |
|---|---|
| (A) | from 1 to 10% by weight of component A, |
| (B) | from 0.5 to 10% by weight of component B, |
| (C) | from 0.5 to 5.0% by weight of component C, |
| (D) | from 0.5 to 8.0% by weight of component D, |
| (E) | from 0.5 to 5.0% by weight of component E, |
| (F) | from 0 to 10% by weight of component F, |
| (G) | from 0 to 5% by weight of component G, |
| (H) | from 0 to 15% by weight of one or more additives |
| | H, and |
| (I) | from 74 to 97.5% by weight water, |
wherein the sum of components A to I is 100% by weight.

5. Composition according to one of Claims 1 to 4, consisting of:
| | |
|---|---|
| (A) | from 1 to 6.0% by weight of component A, |
| (B) | from 1 to 5.0% by weight of component B, |
| (C) | from 1 to 4.0% by weight of component C, |
| (D) | from 1 to 5.0% by weight of component D, |
| (E) | from 0.5 to 2.0% by weight of component E, |
| (F) | from 1 to 5% by weight of component F, |
| (G) | from 0.5 to 3% by weight of component G, |
| (H) | from 5 to 10% by weight of one or more additives H, |
| (I) | from 40 to 89% by weight water, |
wherein the sum of components A to I is 100% by weight.

6. Composition according to one of Claims 1 to 5, **characterized in that** component A is selected from the group consisting of acyl glycinate, its salts and mixtures thereof.

7. Composition according to one of Claims 1 to 6, **characterized in that** component B consists of one or more C₈-C₂₂ N-methyl-N-acylglucamines.

8. Composition according to Claim 7, **characterized in that** component B consists of at least one C₁₂-C₁₈ N-methyl-N-acylglucamine.

9. Composition according to one of Claims 1 to 8, **characterized in that** component C consists of lauric acid, palmitic acid, stearic acid, their salts or mixtures thereof.

10. Composition according to one of Claims 1 to 9, **characterized in that** component D consists of an oily substance or of a mixture of oily substances from the group of the triglyceride oils, the ester oils, the hydrocarbon oils and silicone oils.

11. Composition according to Claim 10, **characterized in that** component D consists of an oily substance or of a mixture of oily substances from the group of the triglyceride oils.

12. Composition according to one of Claims 1 to 11, **characterized in that** component E is present and consists of sodium lauroyl isethionate, sodium cocoyl isethionate or a mixture thereof.

13. Composition according to one of Claims 1 to 12, **characterized in that** component F is present and consists of sodium lauryl ether sulfate, sodium myreth sulfate, sodium lauryl sulfate, sodium cocoyl sulfate or mixtures thereof.

14. Composition according to one of Claims 1 to 12, **characterized in that** the composition is free of component F.

15. Composition according to one of Claims 1 to 14, **characterized in that** component G is present and consists of cocamidopropyl betaine, lauramidopropyl betaine, coco-betaine or mixtures thereof.

16. Composition according to one of Claims 1 to 15, **characterized in that** one or more additives (H) are present, selected from the group consisting of preservatives, fragrances, dyes, further surfactants which do not fall under the definition of components A to G, cationic polymers, film-forming agents, thickeners and gelling agents, superfatting agents, antimicrobial and biogenic active ingredients, moisture-donating agents, stabilizers, acids, lyes and activity enhancers.

17. Composition according to one of Claims 1 to 16, **characterized in that** the sum of components A to H is from 10 to 30% by weight.

18. Composition according to one of Claims 1 to 17, **characterized in that** the composition is a cosmetic, dermatological or pharmaceutical composition.

19. Use of a composition according to one of Claims 1 to 18 as a shampoo, facial cleanser, shower gel or shower cream.

20. Use of the composition according to one of Claims 1 to 18 for the treatment or care of the skin, of the hair or of the skin and the hair.

## Revendications

1. Composition contenant :
(A) au moins un tensioactif acide N-acyl-aminé en tant que composant A, le composant A étant constitué d'au moins un acide aminé acylé en C₈-C₂₂ et étant choisi dans le groupe constitué par le glycinate d'acyle, l'aspartate d'acyle, le glutamate d'acyle, le sarcosinate d'acyle, leurs sels et leurs mélanges,
(B) au moins une N-méthyl-N-acylglucamine en tant que composant B,
(C) au moins un acide gras en C₈-C₂₂ ou un sel d'acide gras en tant que composant C,
(D) au moins un corps huileux en tant que composant D,
(E) éventuellement au moins un iséthionate d'acyle en tant que composant E,
(F) éventuellement au moins un tensioactif sulfoné anionique en tant que composant F,
(G) éventuellement au moins un tensioactif bétaïne en tant que composant G,
(H) éventuellement au moins un additif en tant que composant H et
(I) de l'eau.

2. Composition selon la revendication 1, contenant :
(A) au moins un tensioactif acide N-acyl-aminé en tant que composant A, le composant A étant constitué d'au moins un acide aminé acylé en C₈-C₂₂ et étant choisi dans le groupe constitué par le glycinate d'acyle, l'aspartate d'acyle, le glutamate d'acyle, le sarcosinate d'acyle, leurs sels et leurs mélanges,
(B) au moins une N-méthyl-N-acylglucamine en tant que composant B,
(C) au moins un acide gras en C₁₂-C₂₂ ou un sel d'acide gras en tant que composant C,
(D) au moins un corps huileux en tant que composant D,
(E) au moins un iséthionate d'acyle en tant que composant E,
(F) éventuellement au moins un tensioactif sulfoné anionique en tant que composant F,
(G) éventuellement au moins un tensioactif bétaïne en tant que composant G,
(H) éventuellement au moins un additif en tant que composant H et
(I) de l'eau.

3. Composition selon la revendication 1, constituée par :
| | |
|---|---|
| (A) | 1 à 10 % en poids du composant A, |
| (B) | 0,5 à 10 % en poids du composant B, |
| (C) | 0,5 à 5,0 % en poids du composant C, |
| (D) | 0,5 à 8,0 % en poids du composant D, |
| (E) | 0 à 5,0 % en poids du composant E, |
| (F) | 0 à 10 % en poids du composant F, |
| (G) | 0 à 5 % en poids du composant G, |
| (H) | 0 à 15 % en poids d'un ou de plusieurs additifs H |
| et | |
| (I) | 74 à 98 % en poids d'eau, |
la somme des composants A à I étant de 100 % en poids.

4. Composition selon la revendication 2, constituée par :
| | |
|---|---|
| (A) | 1 à 10 % en poids du composant A, |
| (B) | 0,5 à 10 % en poids du composant B, |
| (C) | 0,5 à 5,0 % en poids du composant C, |
| (D) | 0,5 à 8,0 % en poids du composant D, |
| (E) | 0,5 à 5,0 % en poids du composant E, |
| (F) | 0 à 10 % en poids du composant F, |
| (G) | 0 à 5 % en poids du composant G, |
| (H) | 0 à 15 % en poids d'un ou de plusieurs additifs H |
| et | |
| (I) | 74 à 97,5 % en poids d'eau, |
la somme des composants A à I étant de 100 % en poids.

5. Composition selon l'une quelconque des revendications 1 à 4, constituée par :
| | |
|---|---|
| (A) | 1 à 6,0 % en poids du composant A, |
| (B) | 1 à 5,0 % en poids du composant B, |
| (C) | 1 à 4,0 % en poids du composant C, |
| (D) | 1 à 5,0 % en poids du composant D, |
| (E) | 0,5 à 2,0 % en poids du composant E, |
| (F) | 1 à 5 % en poids du composant F, |
| (G) | 0,5 à 3 % en poids du composant G, |
| (H) | 5 à 10 % en poids d'un ou de plusieurs additifs H, |
| (I) | 40 à 89 % en poids d'eau, |
la somme des composants A à I étant de 100 % en poids.

6. Composition selon l'une quelconque des revendications 1 à 5, **caractérisée en ce que** le composant A est choisi dans le groupe constitué par le glycinate d'acyle, ses sels et leurs mélanges.

7. Composition selon l'une quelconque des revendications 1 à 6, **caractérisée en ce que** le composant B est constitué d'une ou de plusieurs N-méthyl-N-acylglucamines en C₈-C₂₂.

8. Composition selon la revendication 7, **caractérisée en ce que** le composant B est constitué d'au moins une N-méthyl-N-acylglucamine en C₁₂-C₁₈.

9. Composition selon l'une quelconque des revendications 1 à 8, **caractérisée en ce que** le composant C est constitué d'acide laurique, d'acide palmitique, d'acide stéarique, de leurs sels ou de leurs mélanges.

10. Composition selon l'une quelconque des revendications 1 à 9, **caractérisée en ce que** le composant D est constitué d'un ou d'un mélange de corps huileux du groupe des huiles de triglycérides, des huiles d'esters, des huiles d'hydrocarbures et des huiles de silicone.

11. Composition selon la revendication 10, **caractérisée en ce que** le composant D est constitué d'un ou d'un mélange de corps huileux du groupe des huiles de triglycérides.

12. Composition selon l'une quelconque des revendications 1 à 11, **caractérisée en ce que** le composant E est contenu et est constitué d'iséthionate de lauroyle sodique, d'iséthionate de cocoyle sodique ou de leur mélange.

13. Composition selon l'une quelconque des revendications 1 à 12, **caractérisée en ce que** le composant F est contenu et est constitué de lauryléthersulfate de sodium, de myrethsulfate de sodium, de laurylsulfate de sodium, de cocoylsulfate de sodium ou de leurs mélanges.

14. Composition selon l'une quelconque des revendications 1 à 12, **caractérisée en ce que** la composition est exempte du composant F.

15. Composition selon l'une quelconque des revendications 1 à 14, **caractérisée en ce que** le composant G est contenu et est constitué de cocamidopropylbétaïne, de lauramidopropylbétaïne, de coco-bétaïne ou de leurs mélanges.

16. Composition selon l'une quelconque des revendications 1 à 15, **caractérisée en ce qu'**un ou plusieurs additifs (H) sont contenus, choisis dans le groupe constitué par les conservateurs, les parfums, les colorants, les autres tensioactifs, qui ne sont pas compris dans la définition des composants A à G, les polymères cationiques, les agents filmogènes, les agents épaississants et gélifiants, les agents surgraissants, les agents actifs antimicrobiens et biogènes, les agents hydrologiques, les stabilisateurs, les acides, les bases et les agents renforçant l'action.

17. Composition selon l'une quelconque des revendications 1 à 16, **caractérisée en ce que** la somme des composants A à H est de 10 à 30 % en poids.

18. Composition selon l'une quelconque des revendications 1 à 17, **caractérisée en ce que** la composition est une composition cosmétique, dermatologique ou pharmaceutique.

19. Utilisation d'une composition selon l'une quelconque des revendications 1 à 18 en tant que shampoing, produit de nettoyage pour le visage, bain-douche ou crème pour la douche.

20. Utilisation de la composition selon l'une quelconque des revendications 1 à 18 pour le traitement ou le soin de la peau, des cheveux ou de la peau et des cheveux.
